# EUROPEAN PATENT APPLICATION

(11) **EP 1 336 653 A1**
(43) Date of publication of application: **20.08.2003**
(21) Application number: 01976325.9
(22) Date of filing: 24.10.2001
(51) Int. Cl.: C12N 9/08, C12N 9/02

(54) **ENZYME WITH PEROXIDASE ACTIVITY ISOLATED FROM ARTICHOKE (CYNARA SCOLYMUS, L.), PROCESS FOR ISOLATION AND PURIFICATION AND APPLICATIONS**

(30) Priority: 24.10.2000 ES 200002553
(71) Applicant: Universidad de Murcia, 30003 Murcia (ES)
(72) Inventor: RODRIGUEZ LOPEZ, José Neptuno, 30003 Murcia (ES); LOPEZ MOLINA, Dorotea, 30003 Murcia (ES); TUDELA SERRANO, José Bautista, 30003 Murcia (ES); GARCIA CANOVAS, Francisco, 30003 Murcia (ES)
(74) Representative: Maldonado Jordan, Julia
(86) International application number: ES0100400
(87) International publication number: WO02034898

(57) **Abstract**

The enzyme with peroxidase activity which has been isolated from artichoke (Cynara scolymus, L) is a glycosilated protein which can be obtained through a process that comprises the extraction of said enzyme with peroxidase activity from artichoke tissues, the separation of said enzyme from other enzymes with oxidase activity and which may be present in the vegetable material and, optionally, the partial or homogeneous purification of said enzyme with peroxidase activity. Said enzyme can be used in the oxidation of reductor substrates, as clinical or diagnostic reagent, as marker enzyme in immunoassays, for the preparation of biosensors, as biocatalyst for the production of biotechnological and industrial products and in environmental applications.

## Description

### SCOPE OF THE INVENTION

The invention refers to an enzyme with peroxidase activity isolated from artichoke (Cynara scolymus, L.), to a process for its isolation and purification and to its applications.

### BACKBROUND OF THE INVENTION

Peroxidases (EC 1.11.1.7) are oxidereductases that are to be found widely distributed throughout all the phylogenetic scale and that catalyse the oxidation of a wide number of organic and inorganic substrates, using the oxidizing power of the hydrogen peroxide. In addition to its academic and physiological interest, these enzymes are widely used in clinical laboratories and in industry. From among these, the one extracted from the horseradish root (HRP) is the most used.

Peroxidase is widely used in clinical bio-chemistry. Thus, the tests for the determination and quantification of metabolites such as glucose, uric acid, colesterol or triglycerides in biological fluids use peroxidase as coupled enzyme. It is also used in immunoassays for the detection of such well known viruses as the human immunodefficiency virus (HIV) which causes AIDS or herpesvirus. Peroxidases are also used as biocatalysts for the production of biotechnological and industrial products such as phenolic resins, adhesives, deoxidants, antistatics and magnetic radiation protectors, nutritional colorants and detergent bioactive components.

Another property of the peroxidases has made the Scientific Commission of the European Union define them as one of the proteins of greatest biotechnological interest in the XXI Century, and consists of its application in environmental conservation. In this line, peroxidases can replace some chemical reagents and catalyzers used currently in certain types of industries. As example, some peroxidases can substitute chloride in the paper whitening process during its recycling (North American patent US 5.370.770) and also the formaldehyde (mutagenic and cancerous) used in the manufacturing of phenolic resins (North American patent US 5.112.752). Another application of peroxidases in this section is its use in the treatment of liquid residues or soils contaminated with phenol, aromatic amines, chloride compounds and/or heavy metals (North American patent US 5.178.762).

The great industrial interest of this enzyme has originated the search for new sources of peroxidases that catalyze with greater efficiency a specific process and that are more resistant to inactivation. Thus, the isolation and employment of a soya bean peroxidase has been patented for the formation of phenolic resins (North American patent US 5.491.085). This enzyme is highly temperature resistant what provides it with a great efficiency in this process, being a substitute for formaldehyde. New peroxidases have also been isolated from the *coprinus cinereus* micro-organism (North American patent US 5.116.751).

### SUMMARY OF THE INVENTION

The invention faces, in general, the problem of providing new sources of peroxidase.

The solution provided by this invention is based on the fact that inventors have identified an enzyme with peroxidase activity in artichoke, in particular, in artichoke stems, leaves and bracts. Example I describes the isolation and purification of an enzyme from artichoke bracts that presents peroxidase activity [see Example 3.2]

In a particular embodiment, said artichoke stems, leaves and bracts come from artichoke canning industry wastes, that causes a great amount of wastes. For example, 70% weight of the artichoke flower corresponds to wastes not intended for human consumption. However, these are useful for animal food and are intended for the elaboration of silaged forage. In this case, the invention provides an evaluation of said waste.

Therefore, an object of this invention is constituted by an enzyme with peroxidase activity isolated from artichoke.

An additional object of this invention is constituted by a process for the isolation of said enzyme with peroxidase activity from artichokes.

Another additional object of this invention is constituted by a process for the purification of said enzyme with peroxidase activity.

Another additional object of this invention is constituted by the employment of said enzyme with peroxidase activity in the oxidation of reductor substrates, in Bio-medicine and research, in industrial processes, in the elaboration of biosensors and in environmental applications.

### BRIEF DESCRIPTION OF THE DRAWINGS

Figure 1 shows the absorption spectrum of an enzyme with peroxidase activity isolated from artichoke, purified by means of a process provided by this invention.
Figure 2 is a graph representing the ratio between the reaction pH and the relative activity of an enzyme with peroxidase activity isolated from artichoke, purified by means of a process provided by this invention.
Figure 3 is a graph representing the ratio between the reaction temperature and the relative activity of an enzyme with peroxidase activity isolated from artichoke, purified by means of a process provided by this invention.
Figure 4 is a graph representing the stability of an enzyme with peroxidase activity isolated from artichoke, purified by means of a process provided by this invention, as regards time, at an optimum temperature.
Figure 5 is an outline of the applications of an enzyme with peroxidase activity isolated from artichoke provided by this invention [POD: peroxidase]

### DETAILED DESCRIPTION OF THE INVENTION

The invention provides an enzyme with peroxidase activity isolated from artichoke *(Cynara scolymus,* L.), on occasions named in this description "enzyme of the invention". Said enzyme can be isolated from artichoke stems, leaves and bracts by means of a process that shall be herewith described, and if so desired, partially or homogeneously purified. Fractions containing the enzyme of the invention partially purified may be used in multiple clinical and diagnosis tests, as well as in industrial and bio-technological processes and in environmental applications, whilst fractions containing a more purified enzyme of the invention can be used for more specialised techniques and basic research.

In a particular embodiment, said enzyme of the invention is an enzyme isolated from artichoke bracts, homogeneously purified, that corresponds to the majority isoenzyme present in the artichoke bracts. Said isoenzyme has been characterised by means of the tests described in Example 3. As can be observed, said enzyme is a glycosilated enzyme that, though it shows peroxidase activity, is not a peroxidase ascorbate, it has specificity by reductor substrates. Additionally, it has been observed that it has a molecular weight of approximately 42.000 Dalton, determined by electophoresis, and an isoelectric point (pI) approximately over 9,5.

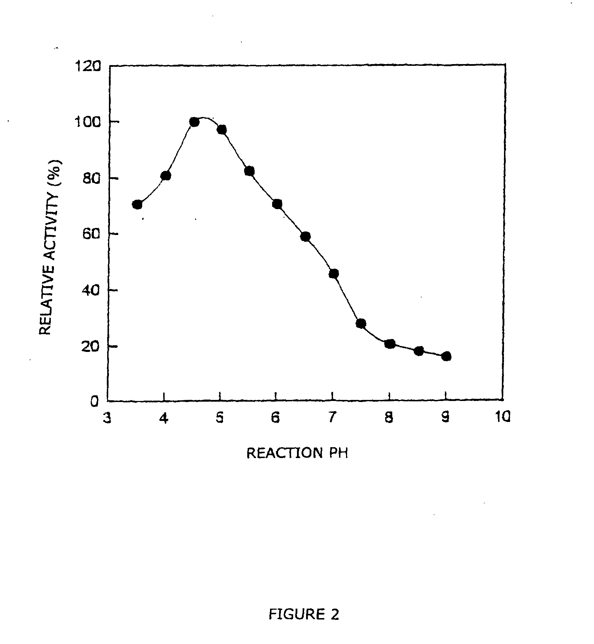

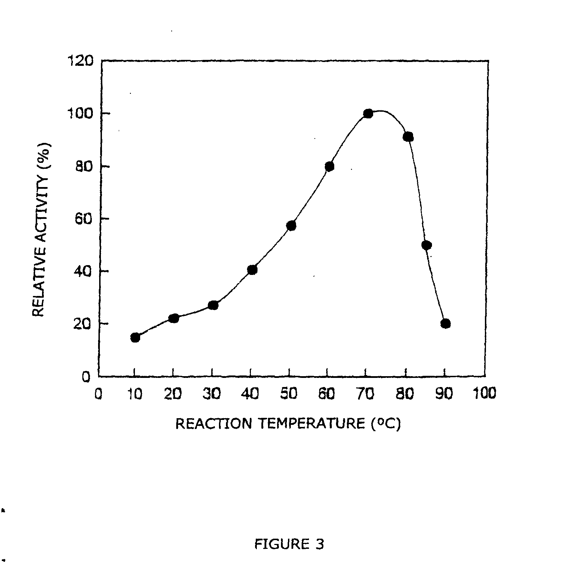

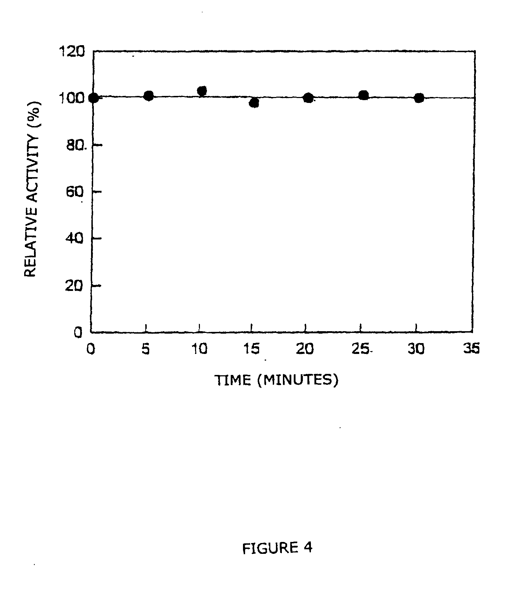

It has also been found that said enzyme has an activity over⁻40% between pH 3,5 and 7 approximately, and an optimum pH at approximately 4,5 (see Figure 2). Likewise, it has been observed, that said enzyme has a relative activity over 40% between approximately 40°C and 85°C, an optimum temperature at approximately 70°C (see Figure 3) and it is stable at the optimum temperature throughout time (see Figure 4).

Said enzyme has shown its capability for oxidizing numerous reductor substrates using the oxidizing power of the hydrogen peroxide, according to the definition of peroxidases (EC 1.11.1.7).

The enzyme of the invention can be obtained from artichoke tissues, in particular, artichoke stems, leaves and bracts, by means of a process that comprises the stages of extraction of said enzyme with peroxidase activity from said artichoke tissues, the separation of said enzyme from other enzymes with oxidase activity eventually present in the vegetable material, and optionally, the partial or homogenous purification of said enzyme with peroxidase activity, that shall be described in detail herewith.

In a particular embodiment of the process for the obtaining of the enzyme of the invention, said artichoke stems, leaves and bracts form part of the artichoke wastes generated by the canning industry, due to which, the process provided by this invention, signifies the extraction and purification of a product of great interest (enzyme with peroxidase activity) from an industrial waste that constitutes a new and economic source of enzyme with peroxidase activity. Moreover, the solid residue obtained after the extraction of said enzyme with peroxidase activity can be used as fibre-enriched material for animal and/or human consumption, or else as a source for the obtaining of other bio-products with sanitary, agricultural-nutritional or industrial applications.

### 1.EXTRACTION

The vegetable artichoke material, including leaves, stem and the external part of the flower (bracts) is subjected to crushing and homogenisation in an aqueous medium constituted by water or by a saline aqueous solution, optionally buffered in a pH interval comprised between 3,0 and 10. Additionally and optionally, said aqueous medium may contain one or more additives selected from among antioxidants, phenol sequesters, cation chelate agents, polymers for phase distribution, organic solvents and/or protease inhibitors or inactivators, that might be present in artichoke tissues. The process as a whole, can be performed within a temperature interval comprised between 4 and 30°C. In a particular embodiment, at 4°C, said aqueous medium contains antioxidants, for example, ascorbic acid and/or phenol sequesters, for example soluble polyvinylpyrrolidone or active carbon, and/or cation chelate agents, for example, 2-hydrate disodium salt ethylendiamine-tetracetic acid (EDTA), polymers for phase distribution such as poliethylenglycol (PEG), dextrans, organic solvents and/or protease inhibitors or inactivators such as 4-(2-aminoethylbenzenesulphonylfluor (AEBSF), bestatin, leupeptin, trans-epoxysuccinyl-L-leucylamide (4-guanidine)butane (E-64), 1,10-phenantroline, pepstatin A, benzamidine and/or phenylmethylsulphonyl fluoride (PMSF).

After homogenization, the soluble part is separated from the solid residue by means of any conventional solid-liquid separation technique, for example, by centrifugation in order to obtain a first soluble fraction (that contains peroxidase activity) and a first solid residue (that retains some of the peroxidase activity). Said first solid residue is subjected to one or more (generally two) additional washing cycles, homogenisation in the previously indicated aqueous medium and solid-liquid separation to obtain a second solid residue, that shall be removed, and second soluble fractions (that contain peroxidase activity). Said second soluble fractions, if so wished, are mixed with said first soluble fraction, constituting the raw extract. After this process, more than 90% of the enzyme with peroxidase activity units is to be found in the soluble fraction or raw extract (the enzyme with peroxidase activity fraction joined to cellular membranes constitutes only 10% of the total activity and can be obtained by extraction assisted by detergents, among them, the non ionic detergents such as Triton X-100 or Triton X-114).

If it is desired, said raw extract, that comprises said first soluble fraction, optionally mixed with said second soluble fractions, is subjected to a washing, desalting and lyophilization process.

On the other hand, by means of a simple washing with water of said second solid residue, a fibre-rich material is obtained, that is free of soluble bio-molecules such as carbohydrates, fats, phenols and proteins, that can be used for manufacturing mixed feed for animals, as a source of fibre in human nutrition and as a source for the obtaining of other bio-products with sanitary, agricultural-nutritional, or industrial applications. Additionally, if so desired, said second solid residue washed with water can be desiccated.

### 2. FRACTIONATION

In plants, in addition to enzymes with peroxidase activity, other enzymes exist with oxidase activity that interfere with the peroxidase when the latter is intended to be used as analysis reagent. One of said enzymes with oxidase activity is the polyphenol oxidase, from now onwards PPO, that is to be found widely distributed in the vegetable world and specifically, in artichoke, where a great concentration of said enzyme exists. Consequently, the separation of PPO and enzymes with peroxidase activity is important for the production of useful fractions of these latter enzymes. The method proposed in this invention aids the separation of said enzymes with the fractionation by means of ammonium sulphate. The PPO precipitates at a low concentration of ammonium sulphate, whilst the enzyme with peroxidase activity is more stable in that salt at the low ammonium sulphate saturation concentrations necessary for the precipitation of the PPO and precipitates at higher saturation concentrations with ammonium sulphate, once the PPO has been separated.

The raw extract obtained during the extraction stage is subjected to fractionation with ammonium sulphate by saturation, in the first place, between 25% and 35%, preferably with 30%, with which it precipitates a fraction containing the totality or most of the PPO. The soluble separated fraction, that contains peroxidase activity, is brought to saturation with ammonium sulphate between 60% and 80%, preferably at 70%, obtaining a precipitation that, washed and dialyzed, has a peroxidase activity and comprises a mixture of isoenzymes with peroxidase activity that contains more than 80% of the initial units and is free from PPO. The extract obtained can be subjected to a purification process to obtain enzyme with the peroxidase activity, partially or homogeneously purified, or else said extract can be desiccated or lyophilised and kept as a source of enzyme with peroxidase activity for assays that do not require the employment of such an enzyme highly purified.

If so wished, the extract containing the peroxidase activity can be washed, desalted and lyophilised.

### 3. PURIFICATION

The enzyme of the invention, in particular, the isoenzyme with majority peroxidase activity, present in artichoke stems, leaves and bracts, can be purified by using any conventional protein purification technique, such as dialysis, ultrafiltration, chromatography, etc. In particular, said isoenzyme can be partially purified by means of chromatographic techniques, with fixed or expanded bed, for example, hydrophobic chromatography (Phenyl Shepharose, Butyl Sepharose, Octyl Sepharose, Phenyl Superose, Alkyl Superose), affinity chromatography (concanavalin A-Sepharose), gel filtration chromatography (Sephadex G-75, G-100, G-200; Sephacryl S-100 HR, S-200HR, S-300HR; Superdex 200; Superose 12), ionic interchange chromatography (DEAE-Cellulose; DEAE-Sepharose; Mono S; Mono Q; Source S; Source Q; CM-Sepharose) or distribution chromatography (Source RPC, Resource RPC) . Additionally, said isoenzyme can be purified homogeneously from the extract obtained in the fractionation stage by means of a combination of said protein purification techniques, in particular of the chromatographic techniques (see Examples 1.3 and 2).

In a particular embodiment, due to the fact that said isoenzyme is a glycoprotein and has a cationic character, in order to purify it homogeneously, the cationic interchange chromatography is performed at a pH comprised in the range of 4,0 and 6,0, preferably at pH 5,0.

In general, all the fractions or extracts with peroxidase activity obtained at any stage of the process for the obtaining of the enzyme of the invention, can be subjected to a washing, desalting and lyophilization process.

The enzyme of the invention can be subjected to immobilisation by means of different processes such as linking to solid supports, entrapment in gels, encapsulation in vesicles, crossing-over between enzyme molecules and the confinement in membrane bioreactors. The glycoproteic character of the enzyme of the invention aids its tie-down to multiple commercial polymers used as immobilisation supports, as for example magnetite or concanavalin-A. This fact, signifies an advantage versus other non glycosilated peroxidases when it comes to its biotechnological application. In any case, the bioreactor could be coupled to filtration, affinity, hydrophobic interaction, ionic interchange, chelatation or distribution systems.

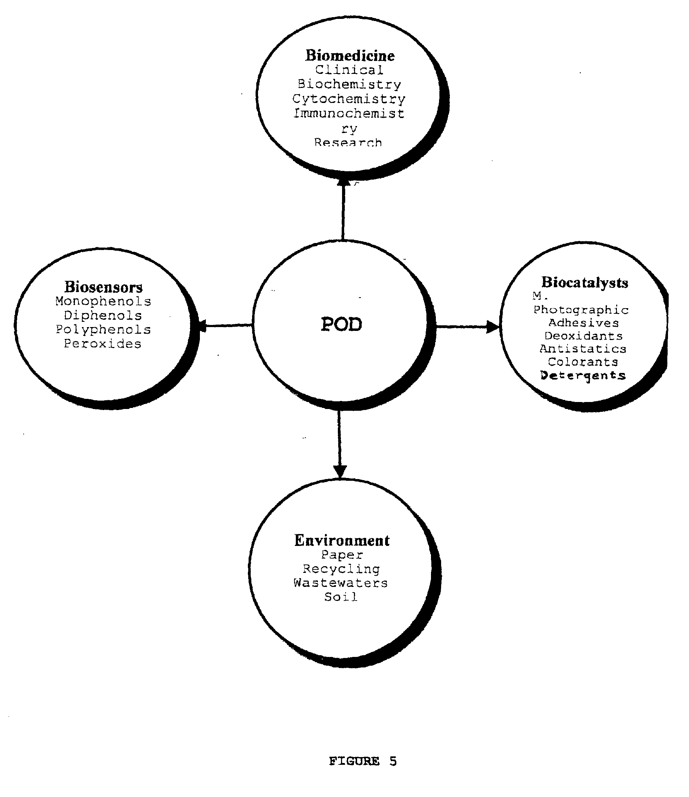

The enzyme of the invention has a structure and activity similar to the peroxidase of the horseradish root, and can be used in any application characteristic to the enzymes with peroxidase activity, as shown in the outline of Figure 5.

Thus, a general application of the enzyme of the invention is its employment in the oxidation of reductor substrates.

The enzyme of the invention can be used, in soluble or immobilised manner, in Biomedicine, for example, in clinical biochemistry, cytochemistry, immuno-chemistry and research, as clinical or diagnostic reagent, for example, in assays for the determination and quantification of metabolites such as glucose, uric acid, colesterol or triglycerides in biological fluids, and as marker enzyme in immunoassays, for example, for the virus detection.

The enzyme of the invention can also be used in industrial applications, for example, as biocatalysts for the production of biotechnological and industrial products, for example, phenolic resins, adhesives, deoxidants, antistatics and magnetic radiation protectors, nutritional colorants and detergent bioactive components.

The enzyme of the invention can also be used in environmental applications, for example, in the whitening of paper during its recycling and in the treatment of liquid residues or soils contaminated with phenol, aromatic amines, chlorate compounds and/or heavy metals.

The enzyme of the invention can also be used in the elaboration of biosensors for the detection of, for example, peroxides, monophenols, diphenols and polyphenols.

The invention, to the extent it can raise the value of a canning industry waste, has additional interest to Agricultural Cooperatives and to the industry related to the transformation of artichoke, since it signifies a commercial option for a scarcely used industrial residue.

The following examples illustrate the invention and shall not be considered as limitative of the scope of the same. Examples 1 and 2, describe the isolation and homogeneous purification of an isoenzyme with peroxidase activity that is majority in artichoke bracts, whilst Example 3 characterises said isoenzyme.

### EXAMPLE 1

### Obtaining of an enzyme with peroxidase activity isolated from artichoke

### 1.1. Extraction

150 grams of artichoke bracts were frozen in liquid nitrogen to aid the crushing and homogenisation of this fibre-rich material. The homogenisation was performed in a 50 mM pH 4,5 acetate buffer containing 30 mM ascorbic acid, 1 M sodium chloride, 1% (p/v) soluble polyvinylpyrrolidone and 10 mM 2-hydrate disodium salt ethylendiaminotetra-acetic acid (EDTA). The extraction buffer contained the following protease inhibitors; pepstatin A (0,005 mM), 1,10-phenantrolin (1 mM) , PMSF (0,1 mM) and benzamidine (1 mM).

After homogenisation, the soluble part was separated from the solid residue by centrifugation (9.000 r.p.m. during 30 minutes). The solid residue was subjected to two more washing, homogenisation and centrifugation cycles in the previous solutions. The floatings of these fractions were joined, constituting the raw extract.

### 1.2 Fractionation

The raw extract obtained during the previous stage (Example 1.1) was brought to 30% saturation with ammonium sulphate. After 1 hour under agitation at 4°C, the extract was centrifuged at 35.000 r.p.m. during 30 minutes and the precipitation, lacking peroxidase activity, was discarded. The floating was brought to 70% saturation with ammonium sulphate and held for 1 hour under agitation at 4°C. After centrifuging at 35.000 r.p.m. during 30 minutes, the precipitate was kept as source of enzyme with peroxidase activity. This precipitate was dissolved in a 20 mM pH 4,5 acetate buffer containing 1 M sodium chloride. The resultant solution was dialysed versus the same buffer, but without sodium chloride, at 4°C all night long. This extract consists of a mixture of isoenzymes with peroxidase activity that contains 80% of the initial units.

### 1.3 Homogeneous purification

From the extract obtained during the previous stage (Example 1.2) an enzyme with peroxidase activity was isolated, with a high degree of purity, by means of a protocol that comprises the following stages:

### 1.3.1 Stage 1

### Hydrophobic chromatography in Phenyl-Shepharose CL-4B

This chromatographic resin was obtained in Pharmacy LKB, and brought to equilibrium with phosphate buffer 50 mM pH 7,0 containing 1,5 M ammonium sulphate. The peroxidase-rich solutions obtained during the previous stage were dialysed all night long versus 2 litres of this same buffer. Approximately 1 ml of resin was used per 3 mg of proteins. After applying the sample at a rate of 2 m1/minutes, the hydrophobic column was eluated at the same rate with a stages gradient of 1,5 M and 0,4 M ammonium sulphate in 50 mM pH 7,0 phosphate buffer, followed by water and 50% polyethylenglycol. Fractions of 7 ml were gathered and tested versus their peroxidase activity and protein concentration. The majority fractions containing peroxidase activity were eluated with 0,4 M ammonium sulphate, joined and concentrated in one microfiltration membrane with a pore-pitch diameter of 10.000 Dalton. Subsequently, this solution was dialysed all night long at 4°C versus 2 litre of a 20 mM pH 5,0 acetate buffer.

### 1.3.2 Stage 2

### Filtration chromatography in Sephacryl S-300 gel

This column was obtained from Pharmacia LKB, coupled to an FPLC system (ÄKTA Explorer 100 of Pharmacia) and brought to equilibrium with 20 nM pH 5,0 acetate buffer. 2 ml of the sample were applied at a rate of 1 ml/minute and fractions of 5 ml were gathered, and tested versus its peroxidase activity. The majority fractions with peroxidase activity flowed in a peak comprised in the range of 38.000 and 46.000 Daltons of molecular weight. These fractions were joined and used for the following purification stage.

### 1.3.3 Stage 3

### Cationic interchange chromatography Resource S

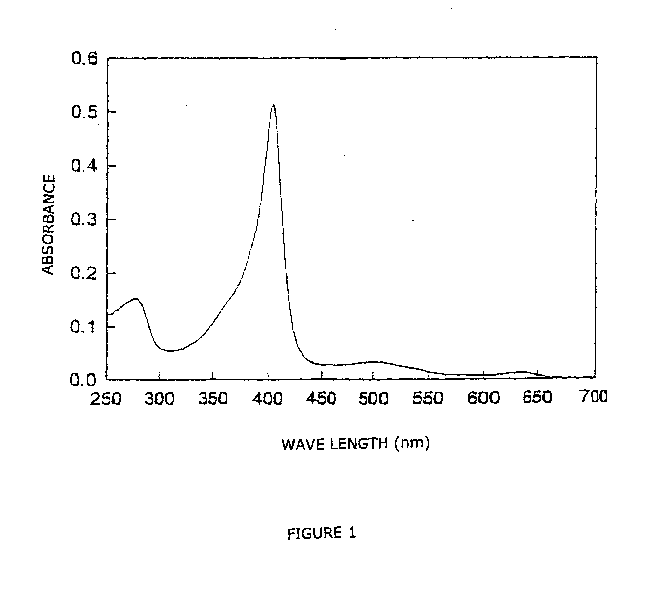

This column was obtained from Pharmacia LKS, coupled to a FPLC system (KTA Explorer 100 of Pharmacia) and brought to equilibrium with 20mM pH 5,0 acetate buffer. After applying the sample at a rate of 2 ml/minute, a lineal gradient was applied from 0 up to 0,3 M of sodium chloride in the same bûffer and fractions of 0,5 ml were gathered. On single peak of peroxidase activity eluated at 0,14 M of sodium chloride. The eluation of this peroxidase was detected by the increase of absorbance at 404 nm (absorbance due to the hemo group) and its activity was determined by using ABTS as substrate. The fractions that presented peroxidase activity were joined, desalted and lyophilised. The absorbance spectrum of this enzyme with peroxidase activity is shown in Figure 1.

The results obtained are shown in Table 1, where the high performance provided by the isolation and purification method proposed by this invention can be observed. Moreover, it can be observed that from a small amount of vegetable material (150 grams) up to 0,5 mg of a pure homogeneous enzyme and with high specific activity, can be obtained. Extrapolating these data at large scale would signify the obtaining of a large amount of peroxidase of a disposable and economic material. Additionally, depending on the degree of purity required, the purification stages could be reduced with the corresponding economic saving.

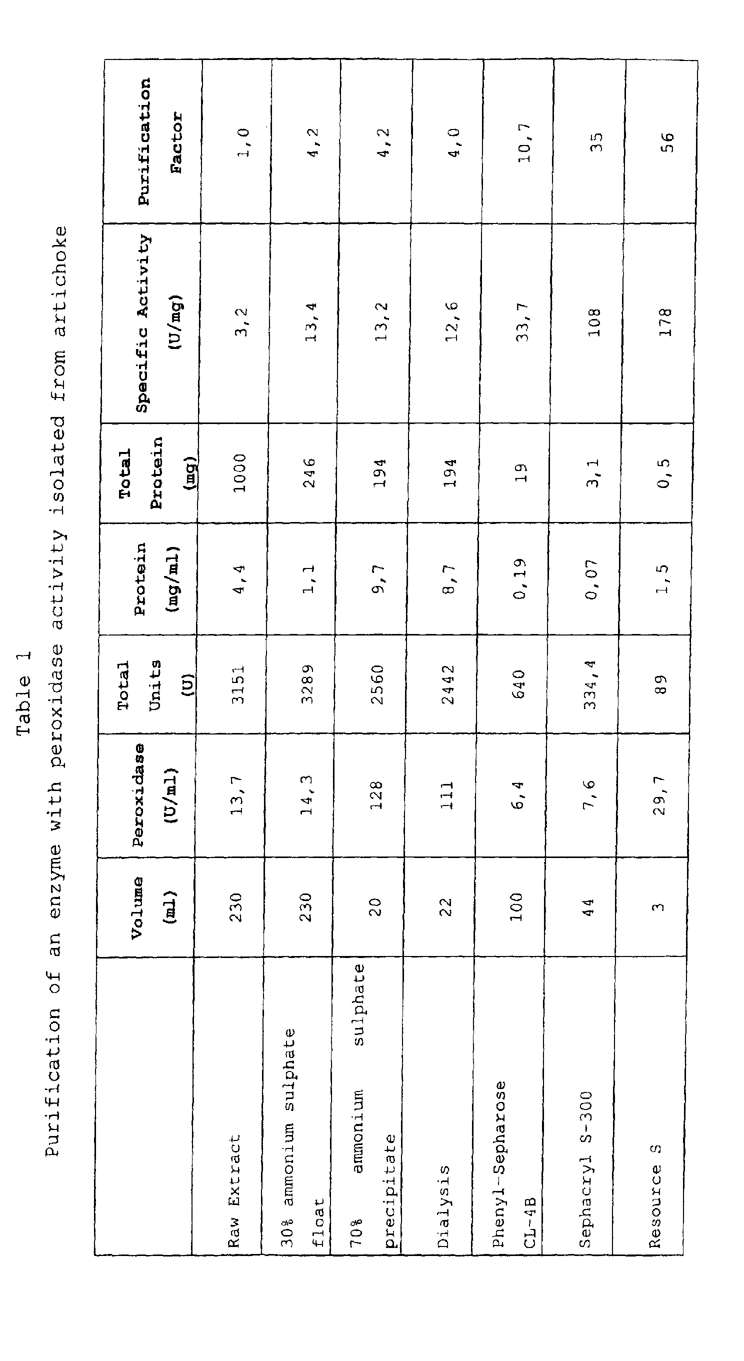

### EXAMPLE 2

### Homogeneous purification of an enzyme with peroxidase Activity isolated from artichoke

This example describes another method to obtain a pure enzyme with peroxidase activity, though using alternative chromatographic stages

### 2.1 Stage 1

### Chromatography in DEAE-Cellulose

This anionic interchanger was obtained from Sigma, packed in a column and brought to equilibrium with Tris-HCl 20 mM pH 8,5 buffer. The peroxidase-rich extract obtained in the extraction phase was injected in said column and was eluated at a rate of 2 ml/minute with a stages gradient of previous buffer free of sodium chloride, 0,1 M and 0,2 M sodium chloride in a Tris-HCl 20 mM pH 8,5 buffer. Approximately 1 ml resin was used per 3 mg of protein. Fractions of 7 ml were gathered and tested versus their peroxidase activity and protein concentration. The majoritary fractions containing peroxidase activity flowed in the fractions not joined to the resin (buffer without sodium chloride), they were joined and concentrated in a microfiltration membrane with a pore-pitch diameter of 10.000 Dalton. Subsequently, this solution was dialysed all night long at 4°C versus a 2 litre Tris-HCl 10 mM pH 7,5 buffer containing 1M sodium chloride and 50mM magnesium chloride.

### 2.2 Stage 2

### Affinity chromatography in A-Shepharose Concanavalin

This column was obtained from Pharmacia LKB, coupled to a FPLC system (ÄKTA Explorer 1 00 Pharmacia) and brought to equilibrium with Tris-HCl 10 mM pH 7,5 buffer containing 1 M sodium chloride and 50 mM magnesium chloride. After applying the sample at a rate of 0,5 ml/minute, the column was eluated with a lineal gradient from 0 up to 0,5 M of methyl-α-D-manopyranoside in the same buffer and fractions of 1 ml were gathered. The eluation of the enzyme with peroxidase activity was detected by the increase of absorbance at 404 nm (absorbance due to the hemo group) and its activity was determined by using ABTS as substrate. The fractions that presented peroxidase activity were joined and dialysed all night long at 4°C versus 2 litres of 20 mM pH 5,4 acetate buffer.

### 2.3 Stage 3

### Cationic interchange chromatography Resource S

This chromatographic stage was performed as in Example 1.3.3 and the performance output was similar to the one observed in Table 1.

### EXAMPLE 3

### Characterisation of the isoenzyme with peroxidase activity majority in artichoke bracts

### 3.1 Purity of the solutions of enzyme with peroxidase activity isolated from artichoke and homogeneously purified

The visible-UV absorption spectrum of the majority isoenzyme with peroxidase activity isolated from artichoke bracts and homogeneously purified by means of the process described in any of the Examples 1 or 2, is shown in Figure 1.

In enzymes with peroxidase activity, the quotient between the absorbance around 400 nm (due to the hemo group) and 280 nm (due to the protein) is known as Rz and is an indication of the purity of the sample of enzyme with peroxidase activity. Thus, those samples of enzyme with peroxidase activity with a Rz comprised between 3,0 and 3,6 are considered pure. The Rz of the protein obtained by means of the homogeneous purification process of the present invention (Examples 1 or 2) is 3,4, which indicates a high degree of purity.

Moreover, this high purity was also observed by means of electrophoresis techniques on polyacrylamide gels under non dissociation (PAGE) and dissociation conditions in the presence of sodium sulphate dodecyl (SDS-PAGE). In fact, the solutions of enzyme with purified peroxidase activity showed one single electroforetic band (PAGE and SDS-PAGE) with a molecular weight of approximately 42.000 Daltons (SDS-PAGE). These results also confirmed the monomeric nature of said enzyme.

### 3.2 Enzymatic activity

The enzyme with peroxidase activity isolated from artichoke provided by this invention, catalyses the oxidation of various compounds in the presence of hydrogen peroxide. The activity of this peroxidase can be determined by the method described as follows, in which the 2,2'-azinebis (3-ethylbenzotiazolin-6-sulphonic) acid (ABTS), for example, is the reductor substrate.

In the first place, 0,1 ml of ABTS 10 mM, 0,1 ml of hydrogen peroxide 5 mM and 0,2 ml of phosphate-citrate 50 mM pH 4,7 *7* buffer were brought to a final reaction volume of 0,95 ml. After bringing the reaction to a temperature of 25°C, 0,05 ml of the enzymatic solution was added and the reaction was followed at 414 nm in a conventional spectrophotometer during 5 minutes. The enzymatic activity was calculated considering a 31.100 M⁻¹cm⁻¹ molar extinction coefficient for the ABTS radical cation (reaction product). A unit (U) of peroxidase activity is the amount of enzyme that oxidises 1 micromol of ABTS per minute. The protein concentration (mg/ml) was determined by means of the Bradford method. The combination of both techniques permitted the calculation of the important specific activity values (U/mg) of the enzyme with peroxidase activity provided by this invention.

### 3.3 Specificity by the reductor substrate

A way of distinguishing between the different classes of peroxidases is to compare their activities versus guaiacol and ascorbic acid.

The enzyme provided by this invention is capable of oxidizing guaiacol at a high rate but did not show activity versus the ascorbic acid, what demonstrates that the majority isoenzyme in artichoke bracts is not a peroxidase ascorbate. Additionally, it demonstrated a high rate of catalysis with reductor substrates such as ABTS, phenol, pyrogallol, catechol, 4-methylcatechol, 4-terc-butylcatechol, 4-hydroxyanisol, p-cresol, p-chloride-phenol or 4-amine-antipyrine, due to which it can be used as coupled enzyme in assays for the determination of metabolites that include some of these reductor substrates. The assays were performed following the protocol described in Example 3.2 but using in each case the reductor substrate to be tested.

### 3.4 Optimum pH

The optimum pH interval at which this enzyme with peroxidase activity shows its maximum activity was examined by using the same reaction medium as the one used in Example 3.2 but substituting the ABTS reductor substrate by guaiacol and using different buffers for the different pH intervals:
30 mM acetate buffer for pH 3,5-5,5;
30 mM phosphate buffer for pH 5,5-8,0;
30 mM Tris-HCl buffer for pH 7,5-9,0 and
30 mM glycine buffer for pH 8,5-9,0;

The results are shown in Figure 2.

### 3.5 Optimum temperature

The activity of this enzyme with peroxidase activity was measured in a temperature interval comprised between 10°C and 90°C and the results are shown in figure 3.

### 3.6 Heat stability

Samples were prepared, adding 0,1 ml of the enzymatic solution to 1,8 ml distilled water previously heated at temperatures comprised between 10°C and 90°C. At each temperature and every 5 minutes, aliquots of 0,05 ml were removed from the enzymatic solution used in the enzymatic activity assays, in compliance with the procedure described in Example 3.2. Figure 4 shows the stability of this enzyme at its optimum temperature.

### 3.7 Other properties

The enzyme with peroxidase activity is a basic or cationic isoenzyme with a pI > 9,5. Moreover, it is glycosilated as is shown by its specific union to concanavalin-A. Thus when 800 U of said enzyme were incubated for 1 hour with 1 ml of Shepharose joined to concanavalin-A at 4°C, 99,9% of the activity was retained by this support. Therefore, this specific union can be used as an alternative purification stage.

## Claims

1. An enzyme with peroxidase activity, **characterised in that** it is obtainable from artichoke *(Cynara scolymus,* L.).

2. Enzyme according to Claim 1, **characterised in that** it is a glycosilated protein.

3. Enzyme according to Claim 1, **characterised in that** it is not a peroxidase ascorbate.

4. Enzyme according to Claim 1, **characterised in that** it has a molecular weight of approximately 42.000 Dalton, determined by electrophoresis.

5. Enzyme according to Claim 1, **characterised in that** it has an isoelectric point greater than approximately 9,5.

6. Enzyme according to Claim 1, **characterised in that** it has an activity over 40% between approximately pH 3,5 and 7.

7. Enzyme according to Claim 6, **characterised in that** it has an optimum pH at approximately 4,5.

8. Enzyme according to Claim 1, **characterised in that** it has a relative activity over 40% between approximately 40°C and 85°C.

9. Enzyme according to Claim 8, **characterised in that** it has an optimum temperature of approximately 70°C.

10. A process for the obtaining of an enzyme with peroxidase activity according to Claim 1, that comprises the extraction of said enzyme with peroxidase activity from artichoke tissues, the separation of said enzyme from other enzymes with oxidase activity, eventually present in the vegetable material, and optionally, the partial or homogeneous purification of said enzyme with peroxidase activity.

11. Process according to Claim 10, in which said artichoke tissues comprise artichoke stems, leaves and bracts.

12. Process according to Claim 11, in which said artichoke stems, leaves and bracts form part of the artichoke wastes generated by the artichoke canning industry.

13. Process according to Claim 10, in which the extraction of said enzyme is performed by means of homogenisation of said artichoke tissues in an aqueous medium, within a temperature interval comprised between 4 and 30°C.

14. Process according to Claim 13, in which said aqueous medium is constituted by water or by a saline aqueous solution, optionally buffered in a pH interval comprised between 3,0 and 10.

15. Process according to Claim 13, in which said aqueous medium additionally includes one or more additives selected from among deoxidants, phenol sequesters, cation chelate agents, polymers for phase distribution, organic solvents and/or protease inhibitors or inactivators and their mixture.

16. Process according to Claim 13, that comprises the crushing of said artichoke tissues prior to the homogenisation of the same.

17. Process according to Claim 13, that comprises the separation, after homogenisation, of the soluble part of the solid residue, by means of a conventional solid-liquid separation process, to obtain a first soluble fraction and a first solid residue.

18. Process according to Claim 17, in which said solid-liquid separation is performed by centrifugation.

19. Process according to Claim 17, in which said first solid residue is subjected to one or more additional washing, homogenisation in aqueous medium and solid-liquid separation cycles, in order to obtain a second solid residue and second soluble fractions that are optionally mixed with said first soluble fraction and constitute a raw extract with peroxidase activity.

20. Process according to any of the Claims 17 or 19, in which said first soluble fraction, optionally mixed with said second soluble fractions, are subjected to a washing, desalting and lyophilization process.

21. Process according to Claim 19, in which said second solid residue is subjected to washing with water to obtain a fibre-rich material free from soluble bio molecules, appropriate for manufacturing mixed feed for animals, as a source of fibre in human nutrition or as a source for the obtaining of other bio-products with sanitary, agricultural-nutritional or industrial applications.

22. Process according to Claim 21, that additionally comprises the desiccating of said second solid residue washed with water.

23. Process according to Claim 10, in which said enzymes with oxidase activity eventually present in the vegetable material comprise the polyphenol oxidase.

24. Process according to Claim 23, in which the separation of the enzyme with peroxidase activity from the polyphenol oxidase is performed by fractionation with ammonium sulphate.

25. Process according to Claim 24, in which the separation of the polyphenol oxidase is performed by precipitation with ammonium sulphate by saturation between 25% and 35%.

26. Process according to Claim 25, in which the separation of the polyphenol oxidase is performed by precipitation with ammonium sulphate by 30% saturation

27. Process according to Claim 24, in which the separation of the enzyme with peroxidase activity from the polyphenol oxidase is performed, once said polyphenol oxidase is separated, by precipitation with ammonium sulphate by saturation between 60% and 80%.

28. Process according to Claim 27, in which the separation of the enzyme with peroxidase activity from the polyphenol oxidase is performed, once said polyphenol oxidase is separated, by precipitation with ammonium sulphate by 70% saturation.

29. Process according to any of the Claims 27 or 28, that comprises the desiccation or lyophilization of the resultant extract and keeping it as a source of enzyme with peroxidase activity for assays that do not require the employment of such an enzyme highly purified.

30. Process according to any of the Claims 27 or 28, that comprises washing, desalting and lyophilization of the resultant extract and keeping it as a source of enzyme with peroxidase activity.

31. Process according to Claim 10, that comprises the total or partial purification of said enzyme with peroxidase activity by means of the employment of one or more conventional protein purification techniques.

32. Process according to Claim 31, that comprises the partial purification of said enzyme with peroxidase activity by means of chromatographic techniques, with fixed or expanded bed, selected from among hydrophobic chromatography, affinity chromatography, gel filtration chromatography, ionic interchange chromatography and distribution chromatography.

33. Process according to Claim 32, in which the partial purification of said enzyme with peroxidase activity is performed by means of affinity chromatography in a concanavalin α-Sepharose column.

34. Process according to Claim 32, in which the partial purification of said enzyme with peroxidase activity is performed by means of cationic interchange chromatography at a pH comprised between 4,0 and 6,0.

35. Process according to Claim 34, in which the cationic interchange chromatography is performed at a 5,0 pH.

36. Process according to Claim 31, that comprises the homogeneous purification of said enzyme with peroxidase activity by means of a combination of two or more chromatographic techniques mentioned in any of the Claims 32 to 35.

37. Process according to any of the Claims 31 to 36, that comprises washing, desalting and lyophilization of the fractions with peroxidase activity.

38. Process according to any of the Claims 29 to 37, that comprises the immobilization of the fractions with peroxidase activity by means of linking to a solid support, entrapment in gels, encapsulation in vesicles, crossing-over of enzyme molecules and confinement in membrane bioreactors.

39. Process according to Claim 38, in which the linking to a solid support is performed on magnetite or concanavalin-A.

40. Process according to any of the Claims 29 to 39, in which the bioreactor that comprises the soluble or immobilized enzyme is coupled to a filtration, affinity, hydrophobic interaction, ionic interchange, chelatation or distribution system.

41. Use of an enzyme with peroxidase activity according to any of the Claims 1 to 9, in the oxidation of reductor substrates.

42. Use of an enzyme with peroxidase activity according to any of the Claims 1 to 9, as clinical or diagnostic reagent, for the determination and quantification of metabolites.

43. Use of an enzyme with peroxidase activity according to any of the Claims 1 to 9, as marker enzyme in immunoassays.

44. Use of an enzyme with peroxidase activity according to any of the Claims 1 to 9, as biocatalyst for the production of biotechnological and industrial products.

45. Use of an enzyme with peroxidase activity according to any of the Claims 1 to 9, in the whitening of paper during its recycling.

46. Use of an enzyme with peroxidase activity according to any of the Claims 1 to 9, in the treatment of liquid residues or soils contaminated with phenols, aromatic amines, chloride compounds and/or heavy metals.

47. Use of an enzyme with peroxidase activity according to any of the Claims 1 to 9 in the elaboration of biosensors for the detection of peroxides and mono-, di- or polyphenols.
